# EUROPÄISCHE PATENTSCHRIFT

(11) **EP 1 761 182 B1**
(45) Veröffentlichungstag und Bekanntmachung des Hinweises auf die Patenterteilung: **06.04.2011**
(21) Anmeldenummer: 04738052.2
(22) Anmeldetag: 30.06.2004
(51) Int. Cl.: A61B 17/72

(54) **CHIRURGISCHER NAGEL**
SURGICAL NAIL
CLOU CHIRURGICAL

(43) Veröffentlichungstag der Anmeldung: 14.03.2007
(73) Patentinhaber: Synthes GmbH, 4436 Oberdorf (CH)
(72) Erfinder: SCHLIENGER, André, CH-4144 Arlesheim (CH); BUETTLER, Markus, CH-4702 Oensingen (CH); SENN, Peter, CH-4437 Waldenburg (CH)
(74) Vertreter: Lusuardi, Werther
(86) Internationale Anmeldenummer: PCT/CH2004/000411
(87) Internationale Veröffentlichungsnummer: WO 2006/002551

(56) Entgegenhaltungen:
- US-A1- 2002 103 488
- US-A1- 2002 173 792

## Beschreibung

Die Erfindung betrifft einen chirurgischen Nagel, insbesondere einen intramedullären Marknagel, gemäss dem Oberbegriff des Patentanspruchs 1.

Die Verriegelung von Marknägeln gehört zum Stand der Technik. Die Einführung der Verriegelungsschrauben oder Verriegelungsbolzen (im folgenden wird nur noch der Begriff Verriegelungsschraube verwendet, der aber auch den Begriff Verriegelungsbolzen mitumfassen soll) in die Queröffnungen des Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Queröffnung ausrichten, sondern weicht davon um einen gewissen Betrag ab. Damit die Verriegelungsschraube trotz dieses Zielfehlers in die Queröffnung mündet und durch diese hindurchgebracht werden kann, wird der Aussendurchmesser der Schraube relativ zum Durchmesser der Queröffnung unterdimensioniert. Bleibt die Zielungenauigkeit im Rahmen dieser Unterdimensionierung, so kann die Verriegelungsschraube trotz des Zielfehlers problemlos durch die Queröffnung geführt werden. Allerdings weist nun die Verriegelungsschraube - wegen der Unterdimensionierung - ein gewisses Spiel auf relativ zur Queröffnung.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmente bewegen können. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube verschieben kann.

Aus der US 2002/173792 HOVER ET AL. ist ein hohler, intramedullärer Marknagel aus Metall bekannt, der in den als Fenster bezeichneten sich diametral gegenüberstehenden Mantelöffnungen der Queröffnung einen oder zwei Kunststoffeinsätze aufweist, durch welche die Verriegelungsschraube eingeführt werden kann. Nachteilig bei diesem bekannten Marknagel ist der Umstand, dass die fensterartigen Kunststoffeinsätze unter den im klinischen Einsatz auftretenden Belastungen eingedrückt werden können, so dass die erwünschte Funktion verloren geht. Aber selbst bei einer sehr vorsichtigen Manipulation dürften die beiden Kunststoffeinsätze beim Durchführen der Verriegelungsschraube aus ihrem "Fenster" gedrückt werden, was ebenfalls zu einem Verlust der Funktion führt.
Bei einer speziellen Ausführungsform von HOVER ist zwar noch zusätzlich zu den beiden Kunststoffeinsätzen in den beiden Fenstern der Queröffnung ein an der distalen Spitze des Marknagels in dessen zentralen Hohlraum einführbaren Einsatz vorgesehen, doch bleibt sein materialmässiger Zusammensetzung und seine Funktion unklar.

Hier will die Erfindung Abhilfe schaffen. Der Erfindung liegt die Aufgabe zugrunde, einen chirurgischen Nagel, insbesondere einen intramedullären Marknagel zu schaffen, mit dem das vorhandene Spiel zwischen ihm und der Verriegelungsschraube risikolos eliminiert werden und eine verbesserte Haltekraft zwischen Verriegelungsschraube und Marknagel erzielt werden kann, ohne dass der Anwender eine erhöhte Präzision bei seiner Arbeit aufbringen muss. Eine weitere Aufgabe besteht darin, einen chirurgischen Nagel mit einem in dessen Längsbohrung einführbaren Einsatz zu schaffen, welcher auch intraoperativ nach der Nagelinsertion ohne grossen Kraftaufwand in diesen einbringbar und ausrichtbar ist, ohne dass dadurch die Haltekraft des eingeführten Einsatzes reduziert wird.

Die Erfindung löst die gestellte Aufgabe mit einem chirurgischen Nagel, welcher die Merkmale des Anspruchs 1 aufweist.

Damit sind folgende Vorteile erzielbar:
a) Der Einsatz wird durch seine radiale Vorspannung axial in der Längsbohrung des Nagels gehalten;
b) das Einschieben des Einsatzes ist ohne eigentliche Spielpassung möglich, so dass beispielsweise keine engen Toleranzen wie bei einem Presssitz eingehalten werden müssen;
c) die Zielgenauigkeit bei der Einbringung der Verriegelungsschraube ist unbeeinträchtigt;
d) Nagel und Einsatz können separat steril verpackt werden und der Chirurg kann wählen ob er den Nagel ohne Einsatz oder mit Einsatz verwenden will. In letzterem Fall kann der Chirurg den Einsatz selbst in Nagel einführen und gegebenenfalls auch wieder entfernen. Verwendet der Chirurg den Nagel ohne Einsatz so bleibt dieser weiterhin steril verpackt für eine nächste. Verwendung. Der Arzt kann somit noch intraoperativ wählen, ob er eine winkelstabile Verriegelung der Verriegelungsschraube einsetzen will oder nicht, wobei der Begriff ,,winkelstabil" eine Einschränkung gewisser Freiheitsgrade bedeutet; und
e) Möglichkeit der winkelstabilen Fixierung des Knochenfragments in gewissen Richtungen für einen bestimmten Betrag der Last.
f) Formschlüssige Arretierung des Einsatzes in der Längsbohrung des Nagels und somit eine erhöhte Haltekraft in der Längsbohrung, was auch bei der Nagelextraktion vorteilhaft ist;
g) leichtes Einbringen und Ausrichten des Einsatzes intraoperativ und nach der Nagelinsertion bei gleichbleibenden Vorteilen bezüglich der Haltekraft;
h) die Erhebungen auf dem Einsatz optimieren die Ausrichtbarkeit und die Haltekraft sowie das Verbleiben des Einsatzes in der Kannulierung bei der allfälligen Extraktion des Nagels; und
i) der längsgeschlitzte Einsatz ermöglicht dessen Einschieben in den kannulierten Marknagel, da der Überstand der Erhebungen, sowie die Grösse des Durchmessers des Einsatzes radial flexibel ist, bei geringerem Haltekraftverlust.

Vorzugsweise ist der Einsatz einstückig ausgebildet. Der Längsschlitz verläuft vorteilhafterweise durchgehend über die gesamte Länge des Einsatzes. Der Einsatz kann aber auch mehrere, nicht über die gesamte Länge des Einsatzes verlaufende Längsschlitze aufweisen, wodurch sich eine erhöhte Stabilität des Einsatzes ergibt. Zudem können die Längsschlitze auch auf dem Umfang des Einsatzes versetzt angeordnet werden, was eine erhöhte Flexibilität ergibt. Die Längsschlitze können zudem auch axial übereinander angeordnet sein.

Bei einer besonderen Ausführungsform ist mindestens eine Erhebung gleich angeordnet wie die mindestens eine Queröffnung und mit dieser in Eingriff bringbar. Die mit den Queröffnungen korrespondierenden Erhebungen unterstützen dabei die Verspannung der Verriegelungsschrauben bei deren Montage.

Die mindestens eine Erhebung ist gegenüber dem mindestens einen Längsschlitz vorzugsweise um 90° versetzt auf dem Umfang des Einsatzes angeordnet. Dadurch ergibt sich der Vorteil, dass beim radialen Komprimieren des Einsatzes die Erhebungen den Durchmesser des Einsatzes nicht überragen, so dass dieser einfach in die Längsbohrung im Nagel eingeschoben werden kann.
Die Höhe der mindestens einen Erhebung ist vorzugsweise kleiner oder gleich der Breite des Längsschlitzes.
Der Längsschlitz kommuniziert vorzugsweise mit der Längsbohrung.

Bei einer besonderen Ausführungsform weist der Nagel mindestens zwei Queröffnungen vorzugsweise mindestens drei Queröffnungen auf. Bei einer weiteren Ausführungsform weist der Nagel mindestens zwei Queröffnungen in seiner distalen Hälfte und mindestens zwei Queröffnungen in seiner proximalen Hälfte auf.

Eine bevorzugte Weiterbildung der Erfindung besteht darin, dass der Einsatz stabförmig ausgebildet ist und durch die Längsbohrung des Nagels bis in den Bereich der Queröffnungen einführbar ist. Der Chirurg kann den Einsatz auch noch nach erfolgter Implantation des Nagels (ohne Einsatz) einsetzen, indem der Einsatz von proximal in die Längsbohrung bis in den Bereich der Queröffnungen vorschiebt.

Das Elastizitätsmodul "e" des Einsatzes beträgt vorzugsweise "e" < 0,8 E und typischerweise "e" < 0,7 E .

Bei einer besonderen Ausführungsform besteht das Material m des Einsatzes aus einem biokompatiblen Kunststoff, vorzugsweise einem Polyethylen oder einem hochmolekulares Polyethylen (HMWPE) . Dies hat den Vorteil, dass kein Abbau des Kunststoffs mit unbekannten Abbauprodukten erfolgt.

Bei einer Alternative besteht das in die Längsbohrung des hohlen Nagels eingebrachte Material geringerer Härte aus einem bioresorbierbarem Kunststoff, welcher vorzugsweise ein Polylactid ist. Bei dieser Ausführung resultiert anfänglich eine spielfreie Querverriegelung des Marknagels, welche dann mit zunehmender Resorption des Polymers sukzessive wieder aufgehoben wird, so dass die Querverriegelungsschraube relativ zum Marknagel und somit auch die versorgten Knochenfragmente wieder beweglich werden. Es erfolgt somit eine Dynamisierung der Knochenfragmente nach erfolgter Frakturkonsolidierung.
Ein weiterer Vorteil des bioresorbierbaren Materials besteht darin, dass die beim Hindurchschrauben einer Verriegelungsschraube durch den Nagel entstehenden Späne vom Körper abgebaut werden können.

Bei einer weiteren Ausführungsform besitzt der Nagel mindestens zwei Queröffnungen, vorzugsweise mindestens drei Queröffnungen. Die Queröffnung weist vorzugsweise einen kreisrunden Querschnitt auf, wobei a = b ist. Die Queröffnung kann aber auch als Langloch mit dem Querschnittsprofil F ausgebildet sein, wobei die längere Dimension ,,a" des Langlochs in axialer Richtung des Nagels angeordnet ist.

Das Material ,,m" des Einsatzes weist vorzugsweise auch eine geringere Dichte ρ₁ als das Material M mit der Dichte ρ₂ auf, wobei vorzugsweise ρ₁ < 0,8 ρ₂ ist.

Der Nagel kann eine in die Queröffnung (mit dem Querschnittsprofil F) und durch den Einsatz einführbare Verriegelungsschraube oder einen Verriegelungsbolzen umfassen, dessen Aussengewinde, beziehungsweise dessen gewindeloser Schaft einen Aussendurchmesser ,,d" aufweist, welcher der Bedingung a > d < b gehorcht.

Bei einer weiteren Ausführungsform weist der Einsatz eine zentrale Längsbohrung auf.

Der Durchmesser der Längsbohrung des Nagels in Richtung seiner Zentralachse kann variabel gestaltet sein und die Längsbohrung vorzugsweise einen zirkulären Absatz aufweisen.

Bei einer weiteren Ausführungsform kann der stabförmige Einsatz auch eine radial, quer zu seiner Längsachse verlaufende Vertiefung aufweisen. Dank dieser Vertiefung, kann eine Verriegelungsschraube oder eine Verriegelungsbolzen leichter zentriert und durch den Einsatz gebohrt werden und es entstehen weniger Späne des Materials ,,m".
Der Einsatz kann auch mehrere Vertiefungen aufweisen, welche gleich angeordnet sind wie die Queröffnungen des Nagels.

Bei einer weiteren Ausführungsform kann der Einsatz stabförmig und vorzugsweise konisch ausgebildet sein. Dank dieser Form kann der Einsatz leichter von distal in die Längsbohrung des Nagels eingeführt werden und zudem ist eine Presspassung möglich.

Bei einer weiteren Ausführungsform weist der stabförmige Einsatz und die Wandung des Nagels zusammenwirkende Mittel auf, vorzugsweise in Form einer Nute und einer dazu passenden Erhebung, welche den Einsatz rotativ in eine vorausbestimmte Position relativ zum Nagel festlegt.

Die Erhebungen weisen eine Querausdehnung ,,x" auf, welche vorteilhafterweise im Verhältnis 1 < x/q < 2 steht, wobei ,,q" der Durchmesser des Einsatzes ist. Der Vorteil dieser Ausführung besteht darin, dass die Erhebungen beim Einführen des Einsatzes in die Längsbohrung des Nagels in die Queröffnungen einschnappen, so dass der Einsatz eindeutig und sicher im Nagel positioniert ist. Im weiteren führt das erhöhte Verdrängungsvolumen zu einer verbesserten Haltekraft, d.h. einer erhöhten Winkelstabilität.

Der Nagel kann bereits mit einem in seine Längsbohrung bis in den Bereich der Queröffnungen eingeführten Einsatz dem Chirurgen zur Verfügung gestellt werden oder alternativ als separat verpackte Teile.

Der Nagel kann zusammen mit einer Verriegelungsschraube mit einem Schraubenschaft und einem Aussengewinde verwendet werden, wobei für den Durchmesser d des Schraubengewindes a > d < b gilt, und ,,d" vorzugsweise mindestens 5 % kleiner ist als die kleinere der beiden Dimensionen a,b.

Bei einer besonderen Ausführungsform ist die Queröffnungen in der distalen Hälfte des Nagels angeordnet.

Falls nur der Nagel nur eine Querbohrung besitzt kann der Einsatz kann in die Längsbohrung bis in den Bereich dieser einzigen Queröffnung eingeführt werden, besitzt der Nagel zwei (oder mehr) Querbohrungen so dann der Einsatz axial bis über die mindestens zwei Queröffnungen eingeführt werden. Dadurch wie eine winkelstabile Fixation des Knochenfragmentes möglich.

Bei einer weiteren Ausführungsform ist der Einsatz axial bis über die mindestens zwei proximalen Queröffnungen eingeführt.

Bei einer bevorzugten Ausführungsform weist der Einsatz n ≥ 2 Erhebungen auf und der Nagel N ≥ n, vorzugsweise N = n Queröffnungen im Bereich des Einsatzes auf.

Zur Herstellung des Nagels kann vom oberen oder unteren Ende des Nagels (aus dem Material M) ein Festkörper aus einem Material ,,m" in die Längsbohrung des Nagels eingeführt werden, so dass der Festkörper mindestens in den Bereich einer der Queröffnungen des Nagels zu liegen kommt.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch einen hohlen Marknagel mit darin eingeführtem radial elastisch komprimierbaren, geschlitzten Einsatz und einer in die Queröffnung einzuführenden Verriegelungsschraube;
Fig. 2 eine perspektivische Ansicht des Einsatzes nach Fig. 1;
Fig. 3 einen Querschnitt durch den Einsatz längs der Linie II-II in Fig. 1;
Fig. 4 eine perspektivische Ansicht eines Einsatzes analog zur Fig. 2;
Fig. 5 eine perspektivische Ansicht eines weiteren Einsatzes analog zur Fig. 2;
Fig. 6 eine perspektivische Ansicht eines weiteren Einsatzes analog zur Fig. 2;
Fig. 7 eine perspektivische Ansicht eines weiteren Einsatzes analog zur Fig. 2;
Fig. 8 eine Seitenansicht des Einsatzes nach Fig. 2 aus biokompatiblem Kunststoff zur Einführung von distal in einen hohlen Marknagel mit radialen Erhebungen, respektiven Durchbohrungen, entsprechend der Position der Queröffnungen im Marknagel;
Fig. 9 einen Längsschnitt durch den Einsatz nach Fig. 8;
Fig. 10 einen Querschnitt des Marknagels im Bereich der Queröffnung mit einem darin eingeführten, sich ausrichtenden Einsatz;
Fig. 11 einen Querschnitt des Marknagels im Bereich der Queröffnung mit einem darin eingeführten, rotationssichernden Einsatz;
Fig. 12 eine perspektivische Ansicht eines Einsatzes aus biokompatiblem Kunststoff zur Einführung von distal in einen hohlen Marknagel mit radialen, rotationssichernden Erhebungen entsprechend der Position der Queröffnungen im Marknagel;
Fig. 13 eine Seitenansicht durch einen Einsatz aus biokompatiblem Kunststoff zur Einführung von proximal in einen hohlen Marknagel;
Fig. 14 eine um 90° gedrehte Seitenansicht durch den Einsatz nach Fig. 13;
Fig. 15 eine Ansicht eines geschlitzten Einsatzes zur Einführung von proximal über die gesamte Länge des hohlen Marknagels;
Fig. 16 eine perspektivische Ansicht eines mit mehreren, radial versetzt angeordneten Längsschlitzen versehenen Einsatzes; und
Fig. 17 eine Seitenansicht des Einsatzes nach Fig. 16.

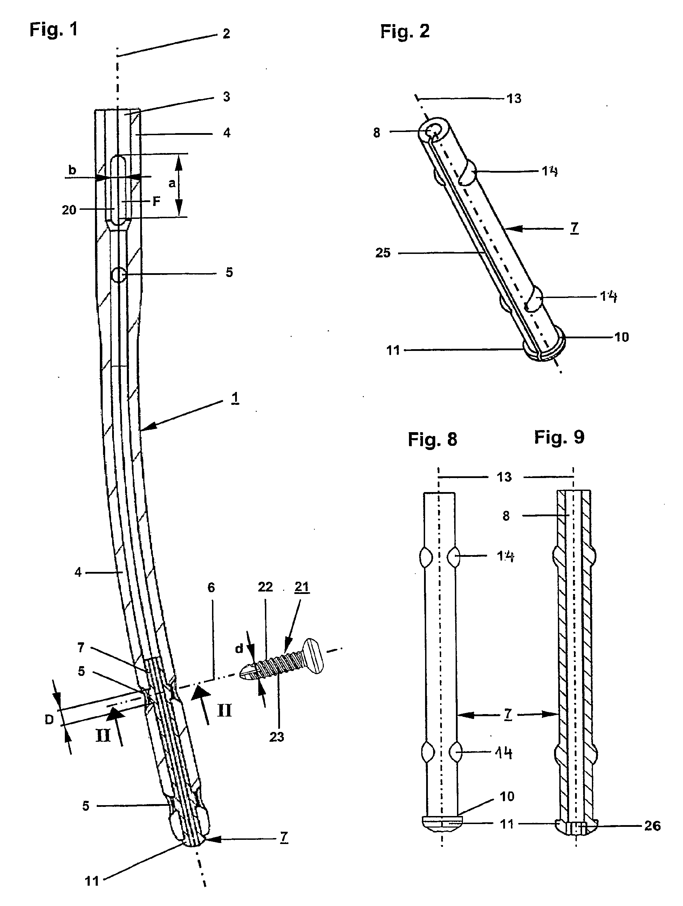

Der in Fig. 1 dargestellte chirurgische Nagel 1 ist ein intramedullärer Marknagel für Röhrenknochen mit einer Zentralachse 2 der aus einem Material M (Metall oder Metallegierung) besteht und drei quer zur Zentralachse 2 verlaufende Queröffnung 5 mit dem Durchmesser D und einer Querachse 6 aufweist.
Eine vierte Queröffnungen ist proximal angebracht und als Langloch 20 ausgebildet, wobei die längere Dimension in axialer Richtung angeordnet ist. Zwei der drei Queröffnungen 5 sind im distalen Teil des Marknagels 1 angebracht.
Der Marknagel besitzt eine koaxial zur Zentralachse 2 verlaufende Längsbohrung 3 und dadurch eine Wandung 4. In diese Längsbohrung 3 ist von distal her ein stabförmiger Einsatz 7 (Fig. 2) in Form eines Festkörpers aus resorbierbarem Polylactid eingesetzt, so dass die Längsbohrung 3 im Bereich der beiden distalen Queröffnungen 5 mit einem Material m geringerer Festigkeit, insbesondere einem geringeren Elastizitätsmodul (im Vergleich zum Material M des Marknagels) passgenau gefüllt ist. Möglich ist aber auch eine Presspassung des Materials m.

Wie in Fig. 1 dargestellt kann eine Verriegelungsschraube 21 mit dem Schaft 22 und dem Aussengewinde 23 in die Queröffnung 5 und damit durch das weichere Material des Einsatzes 7 eingeschraubt werden.

Wie in den Fig. 2-4 dargestellt weist der Einsatz 7 eine koaxial zu seiner Längsachse 13 verlaufende Längsbohrung 8 auf, welche mit einem durchgehenden, über die gesamte Länge des Einsatzes 7 verlaufenden Längsschlitz 25 kommuniziert, so dass der Einsatz 7 radial elastisch komprimierbar ist.
Der Einsatz 7 besitzt an seinem distalen Ende eine halbkugelförmige Erweiterung 11 mit einem nach proximal gerichteten Anschlag 10. Durch den Anschlag 10 der Erweiterung 11 wird eine sichere axiale Positionierung des Einsatzes 7 in der Längsbohrung 3 des Marknagels garantiert. In der halbkugelförmigen Erweiterung 11 ist eine sechskantige Kavität 26 vorgesehen, um einen Sechskant-Schraubendreher aufzunehmen. Der Einsatz 7 weist zudem eine Anzahl von - in die Queröffnungen 5 des Nagels 1 passendem - radialen Erhebungen 14 auf.

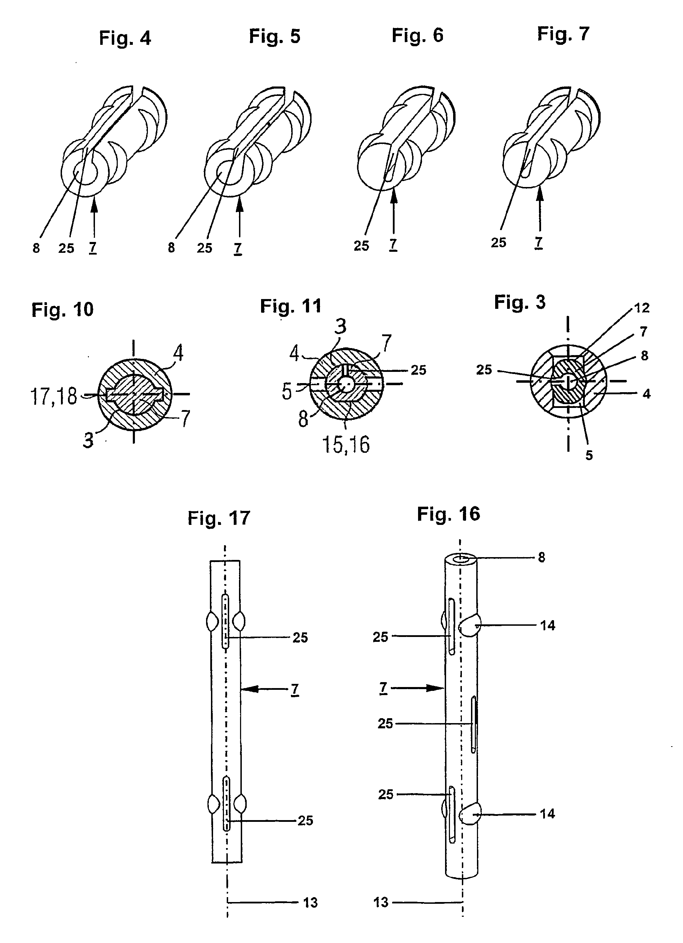

In Fig. 5 ist ein modifizierter Einsatz 7 dargestellt, bei welchem der Längsschlitz 25 asymmetrisch angeordnet ist.

In den Fig. 6 und 7 sind weitere modifizierte Einsätze 7 dargestellt, bei welchen statt einer eigentlichen Längsbohrung 8 mit nach Aussen mündendem Längsschlitz 25 lediglich ein Längsschlitz 25 vorgesehen ist, der entweder neben dem Zentrum (Fig. 6) verläuft oder vorzugsweise (Fig. 7) bis in das Zentrum des Einsatzes einschneidet. Ein solcher Längsschlitz 25 ergibt bereits die erwünschte radiale elastische Komprimierbarkeit des Einsatzes 7.

Der in den Fig. 8 und 9 dargestellt Einsatz 7 kann in Form eines einstückigen Festkörpers aus resorbierbarem Polylactid realisiert werden, so dass die Längsbohrung 3 des Nagels 1 im Bereich der beiden distalen Queröffnungen 5 mit einem Material ,,m" geringerer Festigkeit, insbesondere einem geringeren Elastizitätsmodul ,,e" (im Vergleich zum Material M, bzw. dem Elastizitätsmodul E des Marknagels) passgenau füllbar ist. Möglich ist aber auch eine Presspassung des Materials m.

Wie in Fig. 10 dargestellt, kann der voll ausgebildete Einsatz 7 und die Längsbohrung 3 des Marknagels zwei Rippen/Nuten 17,18 aufweisen, welche eine Rotationsblockierung bewirken.
Wie in Fig. 11 dargestellt, kann der eine Längsbohrung 8 und einen Längsschlitz 25 aufweisende Einsatz 7 und die Längsbohrung 3 des Marknagels Profile 15,16 in Form von Abflachungen aufweisen, welche ebenfalls eine Rotationsblockierung bewirken. Sowohl die Profile 15,16 als auch die Rippen/Nuten 17,18 verlaufen nur über eine Teilstrecke am Frontende des Einsatzes 7.

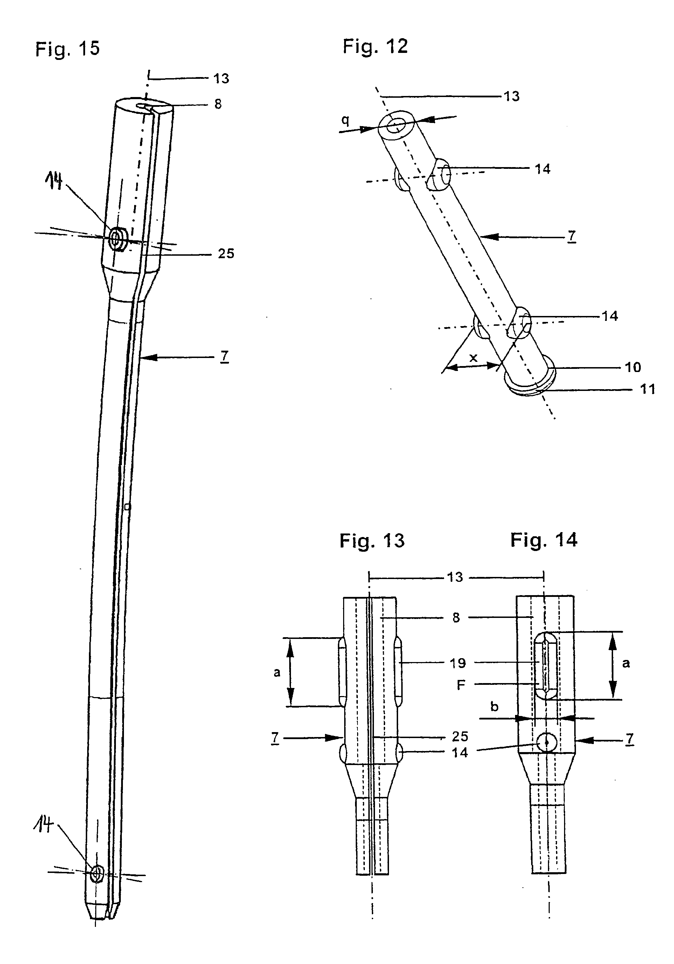

In Fig. 12 ist ein weiterer Einsatz 7 dargestellt, bei welchem die radialen Erhebungen 14 dank ihrer Elastizität in die Öffnungen der Queröffnungen 5 in der Wandung 4 des Nagels 1 einschnappen können, so dass ebenfalls eine axiale und rotative Sicherung des Einsatzes 7 erreicht werden kann. Die Querausdehnung x der Erhebungen 14 steht im Verhältnis 1 < x/q < 2, wobei q der Durchmesser des Einsatzes 7 ist.

In den Fig.13 und 14 ist ein geschlitzter Einsatz 7 dargestellt, der statt von distal, von proximal in die Längsbohrung 3 des Marknagels 1 einführbar ist. Er weist eine axiale Längsbohrung 8 auf, sowie eine zum Langloch 20 (mit dem Querschnittsprofil F mit der Länge a und Breite b) im Marknagel 1 korrespondierende Erhebung 19 auf. Die Erhebung 19 entspricht annähernd der Geometrie des Langlochs 20.

In Fig. 15 ist eine weitere Ausführungsform eines Einsatzes 7 dargestellt, der in etwa die gleiche Länge wie der Marknagel 1 aufweist und somit sämtliche Queröffnungen 5 (Verriegelungsbohrungen) des Marknagels 1 von proximal bis distal abdeckt. Der Einsatz 7 weist eine durchgehende Längsbohrung 8 und einen Längsschlitz 25 auf. Im distalen und im proximalen Teil des Einsatzes 7 ist je eine Erhebung 14 angebracht. Der Einsatz 7 kann bei Bedarf auch noch intraoperativ gekürzt werden.

In den Fig. 16 und 17 ist eine weitere Alternative eines Einsatzes 7 dargestellt, welcher ebenfalls eine Längsbohrung 8 und Erhebungen 14 aufweist. Dieser Einsatz verfügt zudem mehrere, radial versetzt angeordnete Längsschlitze 25.

## Patentansprüche

1. Chirurgischer Nagel (1), insbesondere intramedullärer Marknagel, mit einer Zentralachse (2), der aus einem Material M mit dem Elastizitätsmodul E besteht und mindestens eine quer zur Zentralachse (2) verlaufenden Queröffnung (5) mit dem Querschnittsprofil F und einer Querachse (6) aufweist, wobei das Querschnittsprofil F in Richtung der Zentralachse eine maximale Länge a und senkrecht dazu eine maximale Breite b aufweist, wobei der Nagel (1) eine koaxial zur Zentralachse (2) verlaufende Längsbohrung (3) und eine Wandung (4) aufweist, wobei der Nagel (1) einen über die Längsbohrung (3) in den Bereich der Queröffnung (5) einführbaren longitudinalen Einsatz (7) mit der Längsachse (13) aus einem Material m umfasst, welches ein geringeres Elastizitätsmodul e < E aufweist als das Material M,
**dadurch gekennzeichnet, dass**
A) der Einsatz (7) mindestens einen Längsschlitz (25) aufweist, so dass er radial elastisch komprimierbar ist; und
B) der Einsatz (7) mindestens eine in die Queröffnung (5) passende radiale Erhebung (14) aufweist.

2. Nagel nach Anspruch 1, **dadurch gekennzeichnet, dass** die Queröffnung (5) einen kreisrunden Querschnitt aufweist, wobei a = b ist.

3. Nagel nach Anspruch 1 oder 2, **dadurch gekennzeichnet, dass** der Einsatz (7) einstückig ausgebildet ist.

4. Nagel nach Anspruch einem der Ansprüche 1 bis 3, **dadurch gekennzeichnet, dass** der Längsschlitz (25) durchgehend über die gesamte Länge des Einsatzes (7) verläuft.

5. Nagel nach einem der Ansprüche 1 bis 3, **dadurch gekennzeichnet, dass** der Einsatz (7) mehrere, nicht über die gesamte Länge des Einsatzes (7) verlaufende Längsschlitze (25) aufweist.

6. Nagel nach Anspruch 5, **dadurch gekennzeichnet, dass** die Längsschlitze (25) auf dem Umfang des Einsatzes (7) versetzt angeordnet sind.

7. Nagel nach Anspruch 5 oder 6, **dadurch gekennzeichnet, dass** die Längsschlitze (25) axial übereinander angeordnet sind.

8. Nagel nach einem der Ansprüche 1 bis 7, **dadurch gekennzeichnet, dass** die mindestens eine Erhebung (14) gleich angeordnet ist wie die mindestens eine Queröffnung (5) und mit dieser in Eingriff bringbar ist.

9. Nagel nach einem der Ansprüche 1 bis 8, **dadurch gekennzeichnet, dass** die mindestens eine Erhebung (14) gegenüber dem mindestens einen Längsschlitz (25) um 90° versetzt auf dem Umfang des Einsatzes (7) angeordnet ist.

10. Nagel nach einem der Ansprüche 1 bis 9, **dadurch gekennzeichnet, dass** die Höhe der mindestens einen Erhebung (14) kleiner oder gleich der Breite des Längsschlitzes (14) ist.

11. Nagel nach einem der Ansprüche 1 bis 10, **dadurch gekennzeichnet, dass** der Einsatz (7) eine koaxial zur Längsachse (13) verlaufende Längsbohrung (8) aufweist.

12. Nagel nach einem der Ansprüche 1 bis 11, **dadurch gekennzeichnet, dass** der Längsschlitz (25) mit der Längsbohrung (8) kommuniziert.

13. Nagel (1) nach einem der Ansprüche 1 bis 12, **dadurch gekennzeichnet, dass** er mindestens zwei Queröffnungen (5), vorzugsweise mindestens drei Queröffnungen (5) aufweist.

14. Nagel (1) nach einem der Ansprüche 1 bis 13, **dadurch gekennzeichnet, dass** er mindestens zwei Queröffnungen (5) in der distalen Hälfte des Nagels (1) und mindestens zwei Queröffnungen (5) in der proximalen Hälfte des Nagels (1) aufweist.

15. Nagel (1) nach einem der Ansprüche 1 bis 14, **dadurch gekennzeichnet, dass** er eine, in die das Querschnittsprofil F aufweisende Queröffnung (5) und durch den Einsatz (7) einführbare Verriegelungsschraube oder einen Verriegelungsbolzen (21) umfasst, dessen Aussengewinde (23), beziehungsweise dessen gewindeloser Schaft (22) einen Aussendurchmesser ,,d" aufweist, welcher der Bedingung a > d < b gehorcht.

16. Nagel (1) nach einem der Ansprüche 1 bis 15, **dadurch gekennzeichnet, dass** der Einsatz (7) stabförmig ausgebildet ist.

17. Nagel (1) nach einem der Ansprüche 1 bis 16, **dadurch gekennzeichnet, dass** der Einsatz (7) konisch ausgebildet ist.

18. Nagel (1) nach einem der Ansprüche 1 bis 17, **dadurch gekennzeichnet, dass** der Einsatz (7) und die Wandung (4) zusammenwirkende Mittel aufweisen, vorzugsweise in Form von einer Nute und einer dazu passenden Erhebung, welche den Einsatz (7) rotativ in eine vorausbestimmte Position relativ zum Nagel (1) festlegt.

19. Nagel (1) nach einem der Ansprüche 1 bis 18, **dadurch gekennzeichnet, dass** die Erhebungen (14) eine Querausdehnung ,,x" aufweisen und diese im Verhältnis 1 < x/q < 2 stehen, wobei ,,q" der Durchmesser des Einsatzes (7) ist.

20. Nagel (1) nach einem der Ansprüche 1 bis 19, **dadurch gekennzeichnet, dass** die Queröffnung (5) in der distalen Hälfte des Nagels (1) angeordnet ist.

21. Nagel (1) nach einem der Ansprüche 1 bis 20, **dadurch gekennzeichnet, dass** der Einsatz (7) in die Längsbohrung (3) bis in den Bereich der Queröffnung (5) eingeführt ist.

22. Nagel (1) nach einem der Ansprüche 13 bis 21, **dadurch gekennzeichnet, dass** der Einsatz (7) axial bis über die mindestens zwei Queröffnungen (5) eingeführt ist.

23. Nagel (1) nach einem der Ansprüche 13 bis 22, **dadurch gekennzeichnet, dass** der Einsatz (7) axial bis über die mindestens zwei proximalen Queröffnungen (5) eingeführt ist.

24. Nagel (1) nach einem der Ansprüche 1 bis 23, **dadurch gekennzeichnet, dass** der Einsatz (7) n ≥ 2 Erhebungen (14) aufweist und der Nagel (1) N ≥ n, vorzugsweise N = n Queröffnungen (5) im Bereich des Einsatzes (7) aufweist.

25. Nagel (1) nach einem der Ansprüche 1 bis 24, **dadurch gekennzeichnet, dass** er eine Verriegelungsschraube (21) mit einem Schraubenschaft (22) und einem Aussengewinde (23) umfasst, wobei für den Durchmesser ,,d" des Schraubengewindes (23) die Bedingung a > d < b gilt, und ,,d" vorzugsweise mindestens 5 % kleiner ist als die kleinere der beiden Dimensionen a,b.

## Claims

1. A surgical nail (1), especially an intramedullary nail, with a central axis (2), which consists of a material M with a modulus of elasticity E and has at least one transverse opening (5), which extends transversely to the central axis (2) and has the cross sectional profile F and a transverse axis (6), wherein the cross sectional profile F has a maximum length a in the direction of the central axis and a maximum width b perpendicularly thereto, wherein the nail (1) has a longitudinal borehole (3) and a wall (4) extending coaxially with the central axis (2), wherein the nail (1) comprises a longitudinal insert (7), which can be introduced via the longitudinal borehole (3) into the region of the transverse opening (5), and which has a longitudinal axis (13) and consists of a material m, which has a lower modulus of elasticity e < E than the material M,
**characterized in that**
A) the insert (7) has at least one longitudinal slot (25) so that it can be compressed radially elastically; and
B) the insert (7) has at least one radial elevation (14), which fits into the transverse opening (5).

2. The nail (1) of claim 1, **characterized in that** the transverse opening (5) has a circular cross-section, wherein a = b.

3. The nail (1) of claim 1 or 2, **characterized in that** the insert (7) is constructed in one piece.

4. The nail (1) of one of the claims 1 to 3, **characterized in that** the longitudinal slot (25) extends continuously over the whole length of the insert (7).

5. The nail (1) of one of the claims 1 to 3, **characterized in that** the insert (7) has several longitudinal slots (25), which do not extend over the entire length of the insert (7).

6. The nail (1) of claim 5, **characterized in that** the longitudinal slots (25) are disposed offset over the periphery of the insert (7).

7. The nail (1) of claim 5 or 6, **characterized in that** the longitudinal slots (25) are disposed axially one above the other.

8. The nail (1) of one of the claims 1 to 7, **characterized in that** the at least one elevation (14) is disposed identically with the at least one transverse opening (5) and can be brought into engagement therewith.

9. The nail (1) of one of the claims 1 to 8, **characterized in that** the at least one elevation (14) is disposed on the periphery of the insert (7) offset by 90° with respect to the at least one longitudinal slot (25).

10. The nail (1) of one of the claims 1 to 9, **characterized in that** the height of the at least one elevation (14) is less than or the same as the width of the longitudinal slot (14)

11. The nail (1) of one of the claims 1 to 10, **characterized in that** the insert (7) has a longitudinal borehole (8) extending coaxially with the longitudinal axis (13).

12. The nail (1) of one of the claims 1 to 11, **characterized in that** the longitudinal slot (25) communicates with the longitudinal borehole (8).

13. The nail (1) of one of the claims 1 to 12, **characterized in that** it has at least two transverse openings (5) and preferably at least three transverse openings (5).

14. The nail (1) of one of the claims 1 to 13, **characterized in that** it has at least two transverse openings (5) in the distal half of the nail (1) and at least two transverse opening (5) in the proximal half of the nail (1).

15. The nail (1) of one of the claims 1 to 14, **characterized in that** it comprises a locking screw or a locking bolt (21), which can be introduced into the transverse opening (5) having the cross sectional profile F and through the insert (7) and the external thread (23) or threadless shaft (22) of which have an external diameter "d", which fulfills the condition a > d < b.

16. The nail (1) of one of the claims 1 to 15, **characterized in that** the insert (7) is constructed in the form of a rod.

17. The nail (1) of one of the claims 1 to 16, **characterized in that** the insert (7) is constructed conically.

18. The nail (1) of one of the claims 1 to 17, **characterized in that** the insert (7) and the wall (4) have interacting means, preferably in the form of a groove and an elevation matching said groove, the means fixing the insert (7) rotationally with respect to the nail (1) in a predetermined position.

19. The nail (1) of one of the claims 1 to 18, **characterized in that** the elevation (14) has a transverse extent "x", which fulfills the requirement that 1 < x/q < 2, wherein "q" is the diameter of the insert (7).

20. The nail (1) of one of the claims 1 to 19, **characterized in that** the transverse opening (5) is disposed in a distal half of the nail (1).

21. The nail (1) of one of the claims 1 to 20, **characterized in that** the insert (7) is introduced into the longitudinal borehole (3) as far as the region of the transverse opening (5).

22. The nail (1) of one of the claims 13 to 21, **characterized in that** the insert (7) is introduced axially as far as over the at least two transverse openings (5).

23. The nail (1) of one of the claims 13 to 22, **characterized in that** the insert (7) is introduced axially over the at least two proximal transverse openings (5).

24. The nail (1) of one of the claims 1 to 23, **characterized in that** the insert (7) has n ≥ 2 elevations (14) and the nail (1) has N ≥ n and preferably N = n transverse openings (5) in the region of the insert (7)

25. The nail (1) of one of the claims 1 to 24, **characterized in that** it comprises a locking screw (21) with a screw shaft (22) and an external thread (23), wherein the condition a > d < b applies for the diameter "d" of the screw thread (23) and "d" preferably is at least 5% smaller than the smaller of the two dimensions a, b.

## Revendications

1. Clou chirurgical (1), en particulier clou médullaire centromédullaire, avec un axe central (2) qui est composé d'un matériau M ayant le module d'élasticité E et qui présente au moins une ouverture transversale (5) transversale à l'axe central (2) avec le profil de section transversale F et un axe transversal (6), le profil de section transversale F présentant, en direction de l'axe central, une longueur maximale a et perpendiculairement à cela une largeur maximale b, le clou (1) présentant un alésage longitudinal (3) de façon coaxiale à l'axe central (2) et une paroi (4), le clou (1) comprenant un insert (7) longitudinal pouvant être introduit, via l'alésage longitudinal (3), dans la zone de l'ouverture transversale (5), et ayant l'axe longitudinal (13) réalisé dans un matériau m qui présente un module d'élasticité e < E plus faible que le matériau M,
**caractérisé en ce que**
A) l'insert (7) présente au moins une fente longitudinale (25) de sorte qu'il est comprimable élastiquement dans le sens radial ; et
B) l'insert (7) présente au moins une surélévation (14) radiale s'adaptant dans l'ouverture transversale (5).

2. Clou selon la revendication 1, **caractérisé en ce que** l'ouverture transversale (5) présente une section transversale circulaire, et a = b.

3. Clou selon la revendication 1 ou la revendication 2, **caractérisé en ce que** l'insert (7) est constitué d'une seule pièce.

4. Clou selon l'une des revendications 1 à 3, **caractérisé en ce que** la fente longitudinale (25) court de façon continue sur toute la longueur de l'insert (7).

5. Clou selon l'une des revendications 1 à 3, **caractérisé en ce que** l'insert (7) présente plusieurs fentes longitudinales (25) ne courant pas sur toute la longueur de l'insert (7).

6. Clou selon la revendication 5, **caractérisé en ce que** les fentes longitudinales (25) sont disposées de façon décalée sur la circonférence de l'insert (7).

7. Clou selon la revendication 5 ou la revendication 6, **caractérisé en ce que** les fentes longitudinales (25) sont disposées les unes au-dessus des autres dans le sens radial.

8. Clou selon l'une des revendications 1 à 7, **caractérisé en ce que** la surélévation (14) au moins au nombre de un est disposée de façon identique à l'ouverture transversale (5) au moins au nombre de un et peut être mise en prise avec celle-ci.

9. Clou selon l'une des revendications 1 à 8, **caractérisé en ce que** la surélévation (14) au moins au nombre de un est disposée sur la circonférence de l'insert (7) de façon décalée de 90° par rapport à la fente longitudinale (25) au moins au nombre de un.

10. Clou selon l'une des revendications 1 à 9, **caractérisé en ce que** la hauteur de la surélévation (14) au moins au nombre de un est inférieure ou égale à la largeur de la surélévation (14).

11. Clou selon l'une des revendications 1 à 10, **caractérisé en ce que** l'insert (7) présente un alésage longitudinal (8) qui est coaxial à l'axe longitudinal (13).

12. Clou selon l'une des revendications 1 à 11, **caractérisé en ce que** la fente longitudinale (25) communique avec l'alésage longitudinal (8).

13. Clou (1) selon l'une des revendications 1 à 12, **caractérisé en ce qu'**il présente au moins deux ouvertures transversales (5), de préférence au moins trois ouvertures transversales (5).

14. Clou (1) selon l'une des revendications 1 à 13, **caractérisé en ce qu'**il comprend au moins deux ouvertures transversales (5) dans la moitié distale du clou (1) et au moins deux ouvertures transversales (5) dans la moitié proximale du clou (1).

15. Clou (1) selon l'une des revendications 1 à 14, **caractérisé en ce qu'**il comprend une vis de verrouillage ou un boulon de verrouillage (21) pouvant être introduit(e) dans l'ouverture transversale (5) présentant le profil de section transversale F et à travers l'insert (7), boulon ou vis dont le filet extérieur (23) ou dont la tige sans filet (22) présente un diamètre extérieur « d » qui respecte la condition a > d < b.

16. Clou (1) selon l'une des revendications 1 à 15, **caractérisé en ce que** l'insert (7) est constitué en forme de barre.

17. Clou (1) selon l'une des revendications 1 à 16, **caractérisé en ce que** l'insert (7) est constitué de façon conique.

18. Clou (1) selon l'une des revendications 1 à 17, **caractérisé en ce que** l'insert (7) et la paroi (4) présentent des moyens coopérants, de préférence sous forme d'une rainure et d'une surélévation qui s'y adapte et qui bloque l'insert (7) en rotation dans une position prédéfinie par rapport au clou (1).

19. Clou (1) selon l'une des revendications 1 à 18, **caractérisé en ce que** les surélévations (14) présentent une extension transversale « x », et **en ce que** celles-ci sont dans le rapport 1 < x/q < 2, « q » étant le diamètre de l'insert (7).

20. Clou (1) selon l'une des revendications 1 à 19, **caractérisé en ce que** l'ouverture transversale (5) est disposée dans la moitié distale du clou (1).

21. Clou (1) selon l'une des revendications 1 à 20, **caractérisé en ce que** l'insert (7) est introduit dans l'alésage longitudinal (3) jusque dans la zone de l'ouverture transversale (5).

22. Clou (1) selon l'une des revendications 13 à 21, **caractérisé en ce que** l'insert (7) est introduit dans le sens axial jusqu'au-delà des ouvertures transversales (5) au moins au nombre de deux.

23. Clou (1) selon l'une des revendications 13 à 22, **caractérisé en ce que** l'insert (7) est introduit dans le sens axial jusqu'au-delà des ouvertures transversales (5) proximales au moins au nombre de deux.

24. Clou (1) selon l'une des revendications 1 à 23, **caractérisé en ce que** l'insert (7) présente n ≥ 2 surélévations (14), et **en ce que** le clou (1) présente N ≥ n, de préférence N = n ouvertures transversales (5) dans la zone de l'insert (7).

25. Clou (1) selon l'une des revendications 1 à 24, **caractérisé en ce qu'**il comprend une vis de verrouillage (21) avec une tige de vis (22) et un filet extérieur (23), la condition a > d < b s'appliquant au diamètre « d » du filet de vis (23), et « d » étant de préférence au moins 5 % plus petit que la plus petite des deux dimensions a et b.
